# FASCICULE DE BREVET EUROPEEN

(11) **EP 3 592 753 B1**
(45) Date de publication et mention de la délivrance du brevet: **16.02.2022**
(21) Numéro de dépôt: 18708445.4
(22) Date de dépôt: 09.03.2018
(51) Int. Cl.: C07F 9/113, C07C 67/08, C07C 69/14, C07C 29/68

(54) **NOUVELLE COMPOSITION D'ISOMERES DU 7,9-DODÉCADIÉNYL-1-ACÉTATE ET SON PROCEDE DE FABRICATION**
NEUARTIGE ZUSAMMENSETZUNG VON 7,9-DODECADIENYL-1-ACETAT-ISOMEREN UND VERFAHREN ZUR HERSTELLUNG DAVON
NOVEL COMPOSITION OF 7,9-DODECADIENYL-1-ACETATE ISOMERS AND PROCESS FOR PRODUCTION THEREOF

(30) Priorité: 10.03.2017 FR 1751979
(43) Date de publication de la demande: 15.01.2020
(73) Titulaire: Université de Bordeaux, 33000 Bordeaux (FR); Institut Polytechnique de Bordeaux, 33400 Talence (FR); Centre National de la Recherche Scientifique - CNRS, 75016 Paris (FR)
(72) Inventeur: PUCHEAULT, Mathieu, 33360 Camblanes Et Meynac (FR); LIAUTARD, Virginie, 33130 Begles (FR); GUILLONNEAU, Loic, 64000 Pau (FR); GUERRET, Olivier, 46170 Pern (FR)
(74) Mandataire: Regimbeau
(86) Numéro de dépôt international: PCT/EP2018/055956
(87) Numéro de publication internationale: WO 2018/162739

(56) Documents cités:
- WO-A1-2016/001383
- A Alexakis ET AL: "THE HYDROALUMINATION OF w-TERBUTOXY ALKYNES AN EASY ACCESS TO w-HYDROXY ALKENYL IOOIDES APPLICATION TO THE SYNTHESIS OF OIENIC INSECT PHEROMONES place Jussieu F-75252 PARIS CBdex 05", Tetrahedron Letters, 1 January 1988 (1988-01-01), pages 6243-6246, XP055769628, Retrieved from the Internet: URL:https://doi.org/10.1016/S0040-4039(00) 82315-1
- KUDO EIJI ET AL: "Selective E to Z isomerization of 1,3-Dienes Enabled by A Dinuclear Mechanism", NATURE COMMUNICATIONS, vol. 12, no. 1, 5 March 2021 (2021-03-05), pages 1-8, XP055822291, DOI: 10.1038/s41467-021-21720-4 Retrieved from the Internet: URL:https://www.nature.com/articles/s41467 -021-21720-4.pdf>
- NEGISHI EI-ICHI ET AL: "Palladium-Catalyzed Alkenylation by the Negishi Coupling", CHEMINFORM, vol. 37, no. 38, 19 September 2006 (2006-09-19), XP055822292, ISSN: 0931-7597, DOI: 10.1002/chin.200638271
- CHONG J M ET AL: "Hydroalumination of 3-Butyn-1-ol: Application to a Stereoselective Synthesis of (3E,5Z)-3,5-Dodecadienyl Acetate, the Sex Pheromone of the Leaf Roller Moth", TETRAHEDRON, ELSEVIER SIENCE PUBLISHERS, AMSTERDAM, NL, vol. 55, no. 50, 10 December 1999 (1999-12-10), pages 14243-14250, XP004185350, ISSN: 0040-4020, DOI: 10.1016/S0040-4020(99)00899-6
- Yadav J: "Synthesis of (3E, 5Z)-3,5-Dodecadienylacetate, the Sex Pheromone of Phtheochroa cranaodes (Lepidoptera: Tortricidae)", Bioscience, Biotechnology, and Biochemistry, 1 January 2000 (2000-01-01), pages 1726-1728, XP055822301, Retrieved from the Internet: URL:https://academic.oup.com/bbb/article-p df/64/8/1726/35020297/bbb1726.pdf [retrieved on 2021-07-08]
- Ragoussis Valentine ET AL: "Concise Preparation of the (3 E ,5 Z )-Alkadienyl System. New Approach to the Synthesis of Principal Insect Sex Pheromone Constituents", Journal of Agricultural and Food Chemistry, vol. 52, no. 16, 1 August 2004 (2004-08-01), pages 5047-5051, XP055822295, US ISSN: 0021-8561, DOI: 10.1021/jf049406b
- NEGISHI E I ET AL: "A highly efficient chemo- , regio- , and stereoselective synthesis of (7E, 9Z)-dodecadien-1-yl acetate, a sex pheromone of lobesiabotrana, via a functionalized organoborate", TETRAHEDRON LETTERS, ELSEVIER, AMSTERDAM , NL, vol. 18, no. 5, 1 January 1977 (1977-01-01), pages 411-414, XP027191337, ISSN: 0040-4039 [retrieved on 1977-01-01]
- NEGISHI E I ET AL: "Highly stereoselective syntheses of conjugated E,E- and E,Z-dienes, E-enynes and E-1,2,3-butatriened via alkenylborane derivatives", TETRAHEDRON, ELSEVIER SIENCE PUBLISHERS, AMSTERDAM, NL, vol. 47, no. 3, 1 January 1991 (1991-01-01), pages 343-356, XP026629680, ISSN: 0040-4020, DOI: 10.1016/S0040-4020(01)90494-6 [retrieved on 1991-01-01]
- C R Unelius: "Synthesis and Characterization of the Four Geometrical Isomers of 3,5-Dodecadienyl Acetate", Acta Chemica Scandinavica, 1 January 1998 (1998-01-01), pages 930-934, XP055152125, DOI: 10.3891/acta.chem.scand.52-0930 Retrieved from the Internet: URL:http://actachemscand.org/pdf/acta_vol_ 52_p0930-0934.pdf [retrieved on 2014-11-11]

## Description

### Objet de l'invention :

La présente invention concerne un mélange d'isomères du 7,9-dodécadiényl-1-acétate, dont l'isomère 7E, 9Z est la phéromone sexuelle de l'eudémis de la vigne, ou *Lobesia Botrana,* un lépidoptère ravageur de la vigne. Cette composition est caractérisée en ce que sa pureté isomérique est supérieure ou égale à 98% en l'isomère (7E,9Z) et qu'elle contient au moins 0.1% d'au moins un des isomères (7*Z*,9*E*) et (7Z,9Z), et moins de 1% de l'isomère (7E,9E). Cette composition extrêmement riche en la phéromone de l'eudémis est obtenue selon un nouveau procédé en 2 étapes avec un rendement d'au moins 55%.

Pour des raisons de santé publique et de gestion du potentiel agricole des sols, les technologies de traitement des cultures contre les ravageurs évoluent vers des modes d'action plus ciblés et plus respectueux de l'environnement. A ce titre, l'usage de phéromones sexuelles pour modifier le comportement d'insectes présente des atouts puisque ces phéromones sont spécifiques de chaque espèce de ravageurs et sont efficaces, à des doses très faibles, dans divers types de stratégie (piégeage, confusion sexuelle par exemple).

Cependant un frein au développement de ces technologies réside dans le coût d'accès aux molécules actives. En effet, ces molécules ont souvent de nombreux isomères possibles et les technologies de synthèse sélectives sont généralement coûteuses.

Par ailleurs, le principe d'utilisation des phéromones pour lutter contre des insectes comme l'eudémis de la vigne consiste en particulier à noyer le signal émis par les femelles de l'espèce dans un nuage de phéromone diffusé dans les vignes au moyen par exemple d'un grand nombre de diffuseurs (voir par exemple les systèmes RAK de BASF ou Isonet de Shin-Etsu Chemical). Ce qui garantit l'efficacité de ces diffuseurs est la dose de l'isomère actif par hectare. Or, aujourd'hui, ces diffuseurs sont chargés avec des mélanges d'isomères ne contenant environ que 75% en poids de l'isomère actif. Cela induit une dépense inutile et impose de relarguer dans les champs une quantité inutile de produit chimique. Dans ces perspectives, il est donc important de pouvoir préparer des mélanges d'isomères les plus concentrés possibles en bon isomère.

Enfin, il peut être problématique d'utiliser des mélanges contenant trop d'isomères qui ne sont pas normalement présents dans la phéromone de la lobésia. En effet, ces autres isomères peuvent être des phéromones pour d'autres insectes ce qui détruit l'argument de la sélectivité des produits à base de phéromone.

Le composant principal de la phéromone sexuelle de l'eudémis de la vigne est le (*E*,*Z*)-7,9-dodécadiényl-1-acétate. Cette molécule est porteuse de 2 doubles liaisons et présente donc 4 isomères géométriques possibles rappelés dans le tableau suivant :

**Tableau 1 : isomères géométriques du 7,9-dodécadiényl-1-acétate**

| | |
|---|---|
| 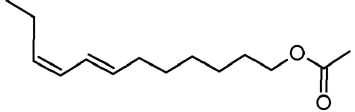 | (E,Z)-7,9-dodécadiényl-1-acétate |
| 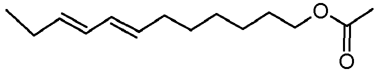 | (E,E)-7,9-dodécadiényl-1-acétate |
| 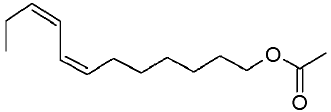 | (Z,Z)-7,9-dodécadiényl-1-acétate |
| 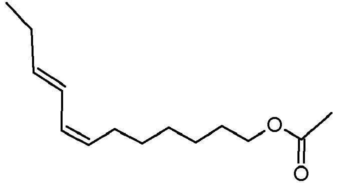 | (Z, E)-7,9-dodécadiényl-1-acétate |

Pour répondre efficacement au problème économique posé par la synthèse de cette phéromone, il convient de prendre en compte les éléments suivants :
- Seul l'isomère (E,Z) est actif. Il est donc essentiel de pouvoir le préparer majoritairement. Parmi tous ces isomères, l'isomère thermodynamiquement le plus stable est l'isomère (E,E).
- Il est important de noter que les 3 isomères autres que le (E,Z) sont connus comme ne gênant pas l'attractivité de la phéromone (Ideses & al. Journal of Chemical Ecology, Vol 8, No. 1, 1982, p. 195)*.*
- L'isomère (E,E) est l'isomère le plus stable et constitue l'impureté inactive principale de toutes les synthèses connues.

De la même façon, un certain nombre d'autres phéromones de nuisibles des cultures comprennent un motif diénique conjugué, et le procédé de la présente invention peut donc leur être appliqué. On peut citer notamment les molécules suivantes : (E,Z)-2,4-Décadiénal, (Z,E)-3,5-Décadiényl acétate, Éthyl (E,Z)-2,4-décadiénoate, Méthyl (E,Z)-2,4-décadiénoate, (E,Z)-3,5-Dodécadiényl acétate, (E,Z)-4,6,10-Triméthyl-2,4-dodécadién-7-one , (E,Z)-5,7-Dodécadién-1-ol, (E,Z)-5,7-Dodécadiénal, (E,Z)-5,7-Dodécadiényl acétate , (E,Z)-7,9-Dodécadién-1-ol, (E,Z)-7,9-Dodécadiénal, (E,Z)-7,9-Dodécadiényl acétate, (E,Z)-8,10-Dodécadién-1-ol, (E,Z)-8,10-Dodécadiénal, (E,Z)-8,10-Dodécadiényl acétate, (Z,E)-3,5-Dodécadiényl acétate, (Z,E)-3,7,11-Triméthyl-2,4,10-dodécatriène, (Z,E)-3,7,11-Triméthyldodéca-2,4-diéne, (Z,E)-5,7-Dodécadièn-1-ol, (Z,E)-5,7-Dodécadiénal, (Z,E)-5,7-Dodécadiényl acétate, (Z,E)-5,7-Dodécadiényl propionate, (Z,E)-7,9-Dodécadién-1-ol, (Z,E)-7,9-Dodécadiényl acétate, (Z, E)-8,10-Dodécadién-1-ol, (Z, E)-8,10-Dodécadiénal, (Z, E)-8,10-Dodécadiényl acétate, (Z,Z)-5,7-Dodécadiénal, (Z,Z)-5,7-Dodécadiényl acétate, (Z,Z)-7,9-Dodécadién-1-ol, (Z,Z)-7,9-Dodécadiényl acétate, (Z,Z)-8,10-Dodécadién-1-ol, (Z,Z)-8,10-Dodécadiényl acétate, (E,Z)-10,12-Tétradécadiényl acétate, acide (E,Z)-3,5-Tétradécadiénoïque, (E,Z)-3,5-Tétradécadiényl acétate, (E,Z)-8,10-Tétradécadiénal, (E,Z)-8,10-Tétradécadiényl acétate, (E,Z)-9,11-Tétradécadiényl acétate, (Z,E)-10,12-Tétradécadiényl acétate, (Z,E)-3,5-Tétradécadiényl acétate, (Z,E)-8,10-Tétradécadièn-1-ol, (Z,E)-8,10-Tétradécadiényl acétate, (Z,E)-9,11-Tétradécadièn-1-ol, (Z,E)-9,11-Tétradécadiénal, (Z,E)-9,11-Tétradécadiényl acétate, (Z,Z)-10,12-Tétradécadièn-1-ol, (Z,Z)-10,12-Tétradécadiényl acétate, acide (Z,Z)-3,5-Tétradécadiénoïque, (Z,Z)-8,10-Tétradécadiénal, (Z,Z)-9,11-Tétradécadién-1-ol, (Z,Z)-9,11-Tétradécadiénal, (Z,Z)-9,11-Tétradécadiényl acétate, (E,Z)-8,10-Pentadécadién-1-ol, (E,Z)-8,10-Pentadécadiényl acétate, (E,Z)-9,11-Pentadécadiénal, (Z,E)-8,10-Pentadécadiényl acétate, (Z,Z)-8,10-Pentadécadiényl acétate, (Z,Z)-9,11-Pentadécadiénal, (E,Z)-10,12-Hexadécadién-1-ol,(E,Z)-10,12-Hexadécadiénal, (E,Z)-10,12-Hexadécadiényl acétate, (E,Z)-11,13-Hexadécadién-1-ol, (E,Z)-11,13-Hexadécadiénal, (E,Z)-11,13-Hexadécadiényl acétate, (E,Z)-9,11-Hexadécadiénal, (E,Z)-9,11-Hexadécadiényl acétate, (Z,E)-10,12-Hexadécadiénal, (Z, E)-10,12-Hexadécadiényl acétate, (Z,Z)-8,10-Heptadécadién-1-ol.

La demande de brevet WO2016001383 décrit une nouvelle voie de synthèse en deux étapes de composés diéniques de formule générale 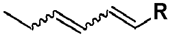 en utilisant un composé intermédiaire de type dialkyl ou diaryl -hexa-1,3-dién-1-yl phosphate de formule générale **2** 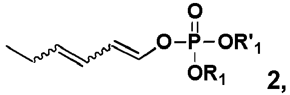 dans laquelle R₁ et R'₁ désignent indépendamment un groupement alkyle ou un groupement aryle.

Ainsi, dans le cas de la synthèse du (E,Z)-7,9-dodécadiényl-1-acétate, cette synthèse en deux étapes permet d'obtenir un mélange final d'isomères composé majoritairement d'isomère (E,Z) typiquement entre 70% et 80%, d'un second isomère majoritaire (E,E) typiquement en proportion de 20% à 30%, et les deux autres isomères en proportions inférieures à 1%.

Un objet de la présente invention est un procédé de synthèse qui permet de produire simplement dans un premier temps un mélange **M1** d'isomères du composé **2** exempt de l'isomère (E,E). Ensuite, dans un second temps une composition **M2** des isomères du 7,9-dodécadiényl-1-acétate présentant une très haute teneur en isomère (E,Z) et de très faibles teneurs des autres isomères peut être obtenue. Typiquement, **M2** présente une pureté isomérique supérieure ou égale à 98% en l'isomère (E,Z) et contient au moins 0.1% des isomères (Z,E) et (Z,Z) et moins de 1% de l'isomère (E,E).

### Etat de l'art :

Du point de vue de la pureté isomérique, l'examen des solutions proposées dans l'art antérieur pour obtenir du 7,9-dodécadiényl-1-acétate conduit aux deux grandes classes de solutions proposées :
Une première classe rassemble les voies de synthèse qui s'attachent principalement à obtenir une grande stéréosélectivité (supérieure à 95%). Généralement, la phéromone (E,Z)-7,9 dodécadiényl-1-acétate est obtenue après un grand nombre d'étapes dont une qui conduit à un intermédiaire final (E,Z) quasiment pur afin d'atteindre la plus grande pureté isomérique possible et dont l'isomère secondaire est l'isomère (E,E). Dans ce type de synthèses, les isomères secondaires (Z,Z), et (Z,E) sont absents.

Dans US 3954818, les auteurs décrivent une synthèse en plus de 9 étapes avec un rendement non précisé et une pureté de la phéromone (E,Z)-7,9-dodécadiényl-1-acétate proche de 99%. Cependant, comme l'intermédiaire clé de ce procédé est le 7-E-dodecen-9-ynol, les auteurs obtiennent naturellement un mélange des isomères (E,Z) et (E,E) pour la phéromone. Par ailleurs, il faut noter que ce procédé est difficilement envisageable industriellement du fait des réactifs utilisés (fil de lithium, butyl lithium, disiamylborane...) et non avantageux du point de vue économique vu le nombre d'étapes.

Dans US 3845108A, le procédé décrit consiste en 8 étapes de synthèse pour un rendement global de 30% à partir de la quatrième étape et une pureté finale de 70% seulement. Le procédé est caractérisé par un intermédiaire iminophosphonate et l'utilisation de réactifs inutilisables industriellement (oxyde de mercure). L'analyse chromatographique du composé final donne la proportion 9 : 1 pour les isomères (E,Z) et (E,E) du 7,9-dodécadiényl-1-acétate.

Dans FR 2341546A1, on retrouve une synthèse en 9 étapes, l'avant dernière étape conduisant à l'obtention du (E,Z)-7,9-dodécadiénol isomériquement pur après une recristallisation à -40°C. Après l'étape finale, le (E,Z)-7,9-dodécadiényl-1-acétate est obtenu avec une proportion de 92% et l'isomère (E,E)-7,9-dodécadiényl-1-acétate avec une teneur de 8% (analyse chromatographique en phase vapeur).

Dans une seconde classe de procédés de synthèse, la pureté isomérique n'est pas la finalité et le pourcentage de pureté isomérique est en général assez faible.

Dans EP 0241335, les auteurs décrivent un procédé en 5 étapes de synthèse avec un rendement global de 10% environ. La pureté isomérique de la phéromone est de 75% au moins car la stéréochimie de la double liaison en position 9 est issue d'une réaction de Wittig impliquant l'ylure de propyl-triphénylphosphonium et conduit à un ratio 75/25 *cis*/*trans* de cette double liaison. L'autre isomère de cette synthèse est donc le (E,E). Ce procédé coûteux nécessite des équipements d'hydrogénation sous pression. Par ailleurs, la réaction de Wittig génère des quantités importantes d'oxyde de triphényl phosphine coûteuses à éliminer.

Le brevet US 4912253 revendique la synthèse de la phéromone de l'eudémis de la vigne par un couplage catalysé au cuivre entre un magnésien (dérivé du chloropentanol) et l'acétate de (E,Z)-2,4-heptadiényle. La préparation du dérivé acétate est cependant difficile et cette voie d'accès bien que convergente reste coûteuse. L'isomère (E,Z)-7,9-dodécadiényl-1-acétate est obtenu avec une pureté de 92% sans autre précision sur la proportion des autres isomères. Cependant, les auteurs expliquent qu'une isomérisation partielle de la liaison Z a lieu pendant la réaction de couplage catalytique en présence d'un catalyseur mixte cuivre/lithium, conduisant donc à la présence de l'isomère (E,E).

Dans US 7932410, une méthode générale pour former des diènes conjugués à longue chaine grasse est décrite, et est caractérisée par l'utilisation d'esters encombrés en position alpha d'une double liaison tels que l'isobutyrate de 1-pentèn-3-yl qui est couplé à un réactif de Grignard via une catalyse à base de complexes du cuivre. Cette méthode n'est pas applicable industriellement à la synthèse du (E,Z)-7,9 dodécadiényl-1-acétate car l'isobutyrate de 1,3-hept-dièn-3-yl nécessaire pour cette synthèse est très difficilement accessible industriellement. De plus, les puretés isomériques ne sont pas données pour le (*E,Z*)-7,9 dodécadiényl-1-acétate dont la synthèse par cette méthode n'est pas décrite. Les autres synthèses conduisent à des puretés isomériques très moyennes.

Dans Alexakis & al. Tetrahedron, vol. 45, n°2, p. 389, 1989, les auteurs décrivent un procédé en 8 étapes qui exploite la réactivité des fonctions époxydes en présence de dérivés du silicium. La sélectivité stéréoisomérique est très élevée, autour de 96.5% en l'isomère (E,Z)-7,9 dodécadiényl-1-acétate. Les auteurs montrent que la double liaison en position 7 ne peut être que trans ce qui implique que seul un mélange d'isomères (E,Z) et (E,E) peut être obtenu par cette voie de synthèse.

Enfin, des méthodes de purification sont couramment utilisées pour enrichir les mélanges obtenus en l'isomère (E,Z), en particulier en diminuant la proportion d'isomère (E,E). Ainsi, la purification à l'urée est souvent citée mais ne conduit pas à des puretés en (E,Z) très importantes.

Une autre technique d'élimination de l'isomère (E,E) est suggérée mais non décrite dans Cassani & al, Tet. Let. 80, 1980, p. 3497, elle consiste à faire réagir le mélange d'isomères sur le tétracyanoéthylène puis de procéder à une séparation sur colonne chromatographique. La nécessité de cette séparation sur colonne rend impropre à l'industrialisation cette méthode.

Finalement, la voie de synthèse la plus courte est donc celle décrite dans WO2016001383 et la demanderesse a découvert un moyen de rendre les procédés de synthèse du 7,9-dodécadiényl acétate, en particulier le procédé décrit dans WO2016001383, sélectifs au point de conduire à un mélange original **M2** des isomères du 7,9-dodécadiényl acétate caractérisé en ce que sa pureté isomérique est supérieure ou égale à 98% en l'isomère (E,Z) et que **M2** contient au moins 0.1% des isomères (Z,E) et (Z,Z) et moins de 1% de l'isomère (E,E).

Le nouveau procédé de la présente invention consiste à faire réagir un énolphosphate de formule **1** avec un diénophile hydrolysable et de constater de manière surprenante que seul l'isomère (E,E) réagit pour donner un adduit qui, après hydrolyse basique, devient soluble dans l'eau et peut être très facilement éliminé. Cette purification de l'intermédiaire clé permet de faire ensuite réagir cet intermédiaire tout en conservant le ratio des isomères.

La demanderesse entend par diénophile hydrolysable un diénophile (notion connue de l'homme du métier) tel que le produit de la réaction de Diels Alder qui peut être facilement transformé en un sel soluble dans une eau par exemple à pH≥8.

Au sens de la présente invention et comme bien connu de l'homme du métier, un diénophile est une molécule, au sens de la réaction de Diels alder, qui possède une double liaison substituée par des groupements appauvrissant la dite double liaison en électron par effet inducteur ou mésomère.

Comme groupements appauvrissant la dite double liaison en électron par effet inducteur ou mésomère, on peut citer les groupements carboxyliques, esters anhydrides, cyano, nitro, sulphonate.

La réaction de Diels-Alder est une réaction chimique utilisée en chimie organique, dans laquelle un alcène (diénophile) s'additionne à un diène conjugué pour former un dérivé de cyclohexène. Dans la réaction de Diels-Alder, les 4 électrons π du diène réagissent avec la double liaison de l'alcène contenant 2 électrons π. Pour cette raison, cette réaction est appelée cycloaddition. La règle d'Aider permet de préciser les conditions qui facilitent la réalisation de ces cycloadditions : la réaction s'effectue plus facilement entre un diène riche en électrons et un diénophile pauvre en électrons. En d'autres termes, un « bon » diène est substitué par des atomes ou des groupes d'atomes donneurs d'électrons, un « bon » diénophile par des atomes ou groupes d'atomes attracteurs (accepteurs) d'électrons.

Lorsque les dits groupements appauvrissant la dite double liaison en électron par effet inducteur ou mésomère présentent par ailleurs la possibilité de se transformer en sels hydrosolubles par action de l'eau dans des conditions basiques, le diénophyle sera qualifié de diénophyle hydrolisable.

Comme diénophile hydrolysable on peut citer par exemple l'acide maléique, l'acide acrylique, l'acide méthacrylique ainsi leurs dérivés hydrolysables comme leurs esters ou anhydrides.

Comme esters on peut citer les acrylates ou méthacrylates de méthyle, d'éthyle de n-propyle ou de n-butyle les esters de maléates comme le diméthyl maléate ou le di éthyl maléate.

Comme anhydrides on peut citer l'anhydride maléique et ses dérivés substitués en position 2 et 3.

Un autre avantage de ce nouveau procédé est que le ratio isomérique obtenu à l'issue de la réaction de l'énolphosphate avec le diénophile hydrolysable est conservé lorsqu'une étape ultérieure est mise en œuvre. Ceci a pour conséquence que le procédé de synthèse du (E,Z) 7,9-dodécadiényl acétate selon la présente invention est bien plus productif que les procédés précédemment décrits, notamment le procédé décrit dans WO2016001383. En effet, dans l'invention précédente, l'énolphosphate contenait 20 à 25% de l'isomère (E,E) indésirable qui consommait une partie du réactif de Grignard en pure perte. Dans la présente invention, la seconde étape est donc 20 à 25% plus productive.

### Description du procédé :

Le premier objet de la présente invention est un procédé de préparation d'un mélange **M1** d'isomères d'énolphosphate de formule **1** 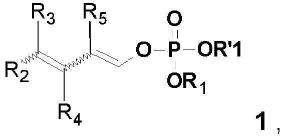 dans laquelle R₁ et désignent indépendamment un groupement alkyle ou un groupement aryle,
R₂ est un groupe alkyle linéaire comprenant de 1 à 8 atomes de carbone
R₃, R₄ et R₅ sont choisis indépendamment parmi H et CH₃,
exempt d'isomère (E,E) et comprenant au moins 98% d'isomère (E,Z), au moins 0.1% d'isomère (Z,Z) et au moins 0.1% d'isomère (Z,E) comprenant les étapes de :
a) Mise en contact d'un mélange d'isomères d'énolphosphate de formule **1** comprenant une quantité détectable d'isomère (E,E) avec un diénophile hydrolysable **D** de préférence dans un solvant organique **S,** à une température **T,** et
b) Hydrolyse basique du milieu obtenu et élimination de l'adduit formé,
c) Caractérisé en ce qu'une quantité détectable est une quantité supérieure à 0.1%,
d) Et que le diénophile hydrolysable est choisi dans le groupe consistant en l'acide maléique, l'acide acrylique, l'acide méthacrylique ainsi que leurs dérivés hydrolysables choisis parmi leurs esters ou anhydrides .

Dans un mode de réalisation particulier, R₁ et désignent indépendamment un groupement alkyle choisi parmi les alkyles en C1-C6, linéaires ou ramifiés et un groupement aryle choisi parmi phényle, benzyle, mésityle, ou tolyle. Le groupement alkyle en C1-C6 linéaire ou ramifié peut être choisi parmi méthyle, éthyle, propyle, iso-propyle, butyle, n-butyle, sec-butyle, iso-butyle, tert-butyle, pentyle, hexyle.

Le mélange d'isomères d'énolphosphate comprenant une quantité détectable d'isomère (E,E) peut être obtenu notamment à partir du (E) hex-2-énal comme décrit dans la demande WO2016001383.

Dans la présente invention, par l'expression « quantité détectable », on entend une quantité supérieure à 0.10%, voire encore supérieure à 0.2% en poids, qui constitue la norme réglementaire de la pharmacopée pour considérer une impureté comme négligeable ou non. Corollairement, une quantité non détectable correspond à une quantité inférieure à 0.2%, voire inférieure à 0.1% en poids.

L'invention vise aussi un procédé pour obtenir un mélange **M2** des isomères d'un composé de formule **3** 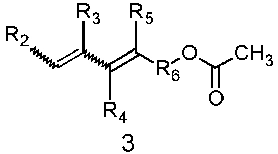 dans laquelle R₂ est un groupe alkyle linéaire comprenant de 1 à 8 atomes de carbone, R₃, R₄ et R₅ sont choisis indépendamment parmi H et CH₃, R₆ représente un groupement alkyle linéaire ou ramifié C₁-C₆ , caractérisé en ce que sa pureté isomérique est supérieure ou égale à 98% en l'isomère (E,Z) et qu'il contient au moins 0.1% des isomères (Z,E) et (Z,Z) et moins de 1% de l'isomère (E,E), comprenant les étapes selon la revendication 1 ou 2, et comprenant en outre une étape c) de mise en contact du mélange **M1** d'isomères d'énolphosphate de formule **1** exempt d'isomère (E,E) et comprenant au moins 98% d'isomère (E,Z), au moins 0.1% d'isomère (Z,Z) et au moins 0.1% d'isomère (Z,E) obtenu à l'étape b) avec un composé de formule XMg-R₆-OMgX, dans laquelle chaque X représente indépendamment un atome d'halogène, et R₆ représente un groupement alkyle linéaire ou ramifié C₁-C₆, puis la mise en contact du mélange obtenu avec un agent d'acylation qui est choisi dans le groupe comprenant les halogénures d'acétoyle, l'anhydride acétique et les acétates d'alkyle tel que l'acétate d'éthyle.

Le procédé selon l'invention peut comprendre en outre une étape c) de mise en contact du mélange **M1** d'isomères d'énolphosphate de formule **1** exempt d'isomère (*E,E*) et comprenant au moins 98% d'isomère (E,Z), au moins 0.1% d'isomère (Z,Z) et au moins 0.1% d'isomère (Z,E) obtenu à l'étape b) avec un composé de formule XMg-R₆-OMgX, dans laquelle chaque X représente indépendamment un atome d'halogène, de préférence Br ou Cl, en particulier Cl, et R₆ représente un groupement quelconque, de préférence un groupement alkyle linéaire ou ramifié C1-C6, de façon plus préférentielle un groupement alkyle linéaire C1-C6, en particulier un groupement hexyle, puis la mise en contact du mélange obtenu avec un agent d'acylation. Cette étape c) produit un mélange **M2** d'isomères d'un composé de formule **3** comprenant au moins 98% d'isomère (E,Z), au moins 0.1% d'isomère (Z,Z) et au moins 0.1% d'isomère (Z,E). 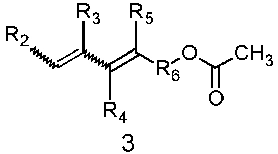 R₂, R₃, R₄, R₅ et R₆ ont les significations telles qu'explicitées plus avant.

De façon préférée, le composé de formule **1** est un composé de formule **2.** 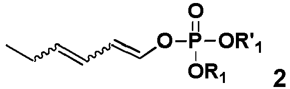 dans laquelle R1 et R'1 désignent indépendamment un groupement alkyle ou un groupement aryle. Ce groupement alkyle peut être choisi parmi les alkyles en C1-C6, linéaires ou ramifiés et ce groupement aryle peut être choisi parmi phényle, benzyle, mésityle, ou tolyle. Ce groupement alkyle en C1-C6 linéaire ou ramifié peut être choisi parmi méthyle, éthyle, propyle, iso-propyle, butyle, n-butyle, sec-butyle, iso-butyle, tert-butyle, pentyle, hexyle.

De façon préférée, la température **T** est supérieure ou égale à 60°C, de préférence supérieure ou égale à 70°C, en particulier égale à 70°C.

De façon préférée, le diénophile hydrolysable **D** est choisi parmi l'acide maléique, l'acide acrylique, l'acide méthacrylique ou leurs dérivés hydrolysables comme leurs esters ou anhydrides. En particulier il s'agit de l'anhydride maléique. Préférentiellement, le diénophile hydrolysable **D** est hydrophile.

Le nombre d'équivalents N de diénophile hydrolysable **D** ajouté dans l'étape a) du procédé selon l'invention peut être n'importe quelle quantité adaptée pour que la réaction se fasse avec le meilleur rendement. Préférentiellement, N est compris entre 0,25 et 10, particulièrement entre 0.5 et 2.

De façon préférée, le solvant organique **S** est un solvant organique ayant un point d'ébullition supérieur à 70°C à pression atmosphérique, tel que les alcanes, les aromatiques ou les solvants polaires. Préférentiellement, **S** est le méthylcyclohexane.

L'étape b) peut être mise en œuvre par ajout, à la solution obtenue à l'issue de l'étape a), de préférence après refroidissement, d'une solution aqueuse basique et récupération des produits organiques par extraction avec un solvant apolaire tel qu'un alcane, un solvant aromatique ou un mélange de ceux-ci.

De façon préférée, l'agent d'acylation est choisi dans le groupe constitué par les halogénures d'acétoyle, l'anhydride acétique et les acétates d'alkyle tel que l'acétate d'éthyle.

L'acétylation est une réaction qui introduit un groupe fonctionnel acétyle dans un composé organique. C'est un cas particulier d'acylation. C'est ainsi le processus d'introduction d'un groupe acétyle (-CO-CH3) sur un composé, pour être précis par substitution d'un atome d'hydrogène actif par un groupe acétyle. L'acétylation de l'hydrogène d'un groupe hydroxyle forme donc un groupe acétoxy : -O-CO-CH3 qui correspond donc à un ester acétate.

Dans un mode de réalisation préféré, le procédé de l'invention, comprend dans une première étape l'obtention du composé énolphosphate de formule **1** sous forme de mélange d'isomères conformément au protocole décrit dans WO2016001383 et schématisé ci-dessous dans le cas de l'énolphosphate de formule 2.

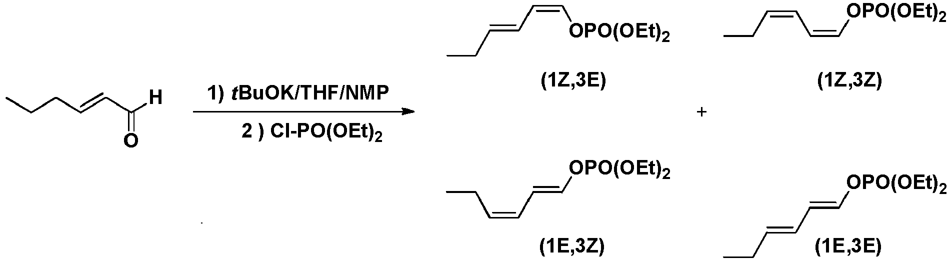

Le mélange obtenu est ensuite lavé avec de l'eau à pH>8 pour éliminer les sels, le chlorophosphate excédentaire et la NMP (N-méthylpyrrolidone).

On ajoute ensuite un solvant organique S ayant un point d'ébullition supérieur à 70°C tel que des alcanes, des aromatiques ou des solvants polaires. Parmi ces solvants on pourra utiliser l'heptane, le méthylcyclohexane, des huiles minérales, le toluène, le xylène, un diméthylbenzène, le méthyl THF, le diméthyl formamide, le diméthyl sulfoxyde. On ajoute alors un nombre **N** d'équivalents, (N compris entre 0,25 et 10, particulièrement entre 0.5 et 2) d'un diénophile hydrolysable **D** tel que l'acide maléique, l'acide acrylique, l'acide méthacrylique ou leurs dérivés hydrolysables comme les esters ou anhydrides, plus particulièrement l'anhydride maléique. De préférence, le diénophile hydrolysable **D** est l'anhydride maléique. Le mélange est alors chauffé à une température **T** supérieure ou égale à 60°C jusqu'à disparition de l'isomère (E,E). La réaction est suivie par les techniques chromatographiques connues de l'homme du métier. Le temps de réaction est fonction du nombre d'équivalents de diénophile, de sa nature et de la température choisie pour la réaction. Après refroidissement, on ajoute une solution basique et on récupère les produits organiques par extraction avec un solvant apolaire tel qu'un alcane ou un solvant aromatique. Cette purification est schématisée sur le schéma ci-dessous dans le cas de l'énolphosphate de formule 2.

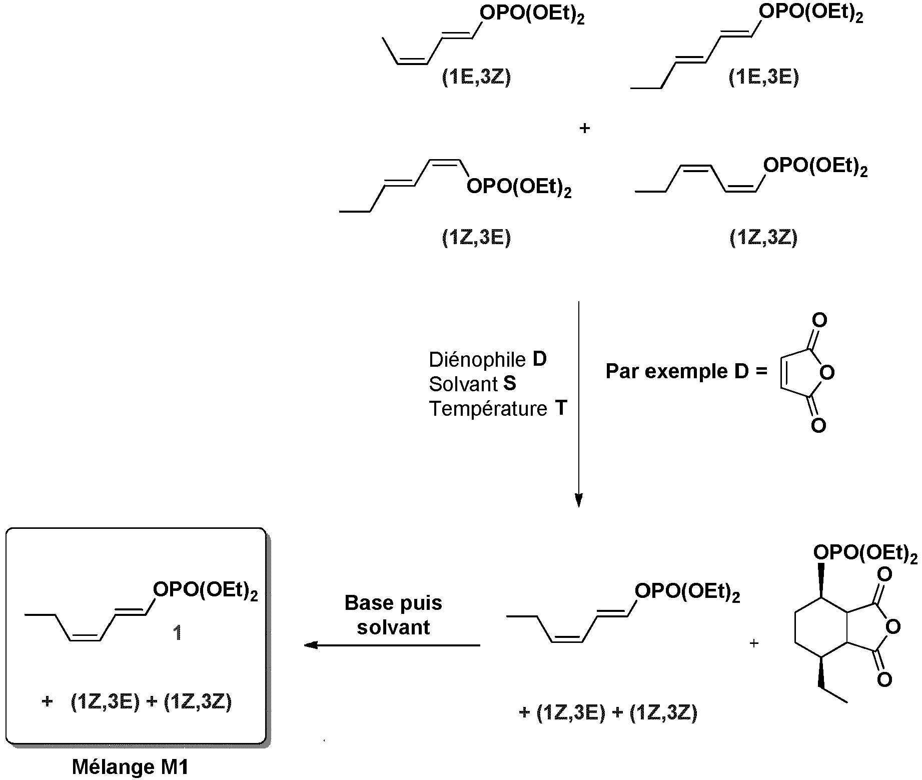

Les fractions extraites sont rassemblées et concentrées sous vide partiel. On obtient alors un mélange M1 des isomères (*E,Z*)*,* (*Z,E*)*,* et (Z,Z) de l'énolphosphate 1 caractérisé en ce que sa pureté isomérique est supérieure ou égale à 98% en l'isomère (E,Z) et que M1 contient au moins 0.1% d'isomère (Z,E) et au moins 0.1% d'isomère (*Z*,*Z*). Ce mélange M1 d'isomères du diéthyl-hexa-1,3-dién-1-yl phosphate est un autre objet de la présente invention.

Un second objet de la présente invention est donc un mélange **M1** d'isomères d'énolphosphate de formule **1** exempt d'isomère (E,E) et comprenant au moins 98% d'isomère (E,Z), au moins 0.1% d'isomère (Z,Z) et au moins 0.1% d'isomère (Z,E).

Par mélange « exempt d'isomère (E,E) », on désigne un mélange dans lequel l'isomère (E,E) n'est pas détectable par les techniques d'analyse classiques connues de l'homme du métier qui permettent de différencier les différents isomères. Par exemple, la technique d'analyse utilisée peut être la résonance magnétique nucléaire RMN, ou la GC (chromatographie en phase gazeuse), de préférence la GC.

De façon générale, dans la présente invention, les proportions des différents isomères et les puretés isomériques peuvent être déterminées par l'homme du métier par toute technique quantitative adaptée, notamment par RMN, HPLC (chromatographie en phase liquide à haute performance) ou GC, en particulier par GC.

Le procédé selon l'invention peut comprendre ensuite une étape consistant à transformer ce mélange d'isomères selon l'étape 2 du protocole décrit dans WO2016001383 pour obtenir un mélange **M2** d'isomères d'un composé de formule **3,** en particulier du 7,9-dodécadiényl-1-acétate, caractérisé en ce que sa pureté isomérique est supérieure ou égale à 98% en l'isomère (E,Z) et que **M2** contient au moins 0.1% des isomères (Z,E) et (Z,Z) et moins de 1% de l'isomère (E,E) par simple évaporation des volatiles. Le mélange **M2** est un autre objet de la présente invention.

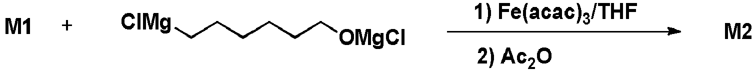

La présente description divulgue aussi un mélange **M2** d'isomères d'un composé de formule **3,** dans laquelle R₂ est tel que défini ci-avant, comprenant au moins 98% d'isomère (E,Z), au moins 0.1% d'isomère (Z,Z) et au moins 0.1% d'isomère (Z,E).

C'est aussi un objet de la présente invention que de fournir un procédé d'obtention du mélange **M2** comprenant la mise en contact du mélange **M1** d'isomères d'énolphosphate de formule 1 selon la présente invention, exempt d'isomère (E,E) et comprenant au moins 98% d'isomère (E,Z), au moins 0.1% d'isomère (Z,Z) et au moins 0.1% d'isomère (Z,E), avec un composé de formule XMg-R₆-OMgX, dans laquelle chaque X représente indépendamment un atome d'halogène de préférence Br ou Cl, en particulier Cl, et R₆ représente un groupement alkyle linéaire ou ramifié en C1-C6, de façon plus préférentielle un groupement alkyle linéaire en C1-C6, en particulier hexyle, puis la mise en contact du mélange obtenu avec un agent d'acylation.

De façon préférée, l'agent d'acylation est choisi dans le groupe constitué par les halogénures d'acétoyle, l'anhydride acétique et les acétates d'alkyle tel que l'acétate d'éthyle.

### Exemples :

Les matières premières et solvants sont les matières premières trouvées commercialement chez Sigma Aldrich.

L'énolphosphate (diéthyl-hexa-1,3-diénylphosphate) est synthétisé en appliquant le procédé décrit dans le brevet WO2016001383.

Le réactif de Grignard est préparé à partir de 6-chloro-hexan-1-ol, de n-Butylmagnesium chloride et de magnésium.

La méthode analytique consiste en une analyse par chromatographie en phase gaz (GC) sur un appareil HP 5890 Series II équipé d'un détecteur FID. La colonne chromatographique est une colonne Innowax 30m, 0.25 mm, 0.25 µm, le gaz vecteur étant l'hélium, et la pression à 0,758423 bar (11 psi).

Le four suit le profil de température suivant: T0=150°C, Temps initial 10 min. Gradient 20°/min ; Température finale : 200°C. Durée 7 min.

L'injecteur est à 250°C, le détecteur à 300°C.

Le volume injecté est de 1 µl. La concentration de l'échantillon est de 4 g/l dans l'acétate d'éthyle (AcOEt).

Les réactions sont réalisées dans un réacteur de 20 I avec agitation mécanique (400 rpm), équipé d'un thermomètre et d'une entrée d'azote. Le système est au besoin refroidi avec un cryostat.

### Exemple 1 (hors invention) : amélioration de la pureté isomérique d'un mélange d'isomères du 7,9-dodécadiényl acétate par traitement à l'urée.

On charge dans un réacteur en verre de 20 I, 2,94 kg (8,92 équiv.) d'urée, et 11,8 I (5,9 vol) de méthanol puis on lance l'agitation tout en maintenant la température à 55 ± 5°C jusqu'à complète dissolution des solides.

On ajoute 2065 g (1 équiv.) d'un mélange d'isomères du 7,9-dodécandiényl acétate de façon à ce que la température interne du réacteur ne descende pas en dessous de 40°C. La température est maintenue à 40 ± 5°C pendant 1 heure, puis descendue à 20 ± 5°C en 3 heures. La cristallisation s'initialise pendant ce refroidissement. On agite ensuite le mélange réactionnel à 20± 5°C pendant plus de 12 h, puis on continue à descendre la température de 5°C par heure jusqu'à atteindre une température de 5°C que l'on maintient pendant 2,5 h tout en continuant à agiter.

On enlève les solides par filtration sous vide du mélange réactionnel. Les produits sont ensuite lavés au méthanol (500 ml) et concentrés par filtration sous vide, puis lavés sous agitation avec un mélange eau (2 vol.) et MTBE (1,15 vol.). Après séparation des phases, on enlève la phase aqueuse et on concentre la phase organique. On obtient 1334 g d'un nouveau mélange d'isomères du 7,9-dodécadiényl acétate, soit un rendement de 65 %.

Après cette première inclusion à l'urée de 2065 g du mélange d'isomères de la phéromone, qui conduit à 1334 g de phéromone enrichie, une deuxième inclusion à l'urée est effectuée dans un réacteur de 1I, avec 100 g du lot enrichi (même protocole que celui décrit précédemment en gardant la proportionnalité des agents de réaction). Une troisième inclusion est ensuite menée avec 62.6g du mélange d'isomères de la phéromone doublement enrichie et toujours le même protocole.

La pureté isomérique des produits, après une, deux et trois inclusions à l'urée, est caractérisée par chromatographie gazeuse on obtient le résultat présenté dans le tableau ci-dessous.

| | Isomère (7*Z*,9*Z*) | Isomère (7*E*,9*Z*) | Isomère (7*Z*,9*E*) | Isomère (7*E*,9*E*) |
|---|---|---|---|---|
| Mélange initial d'isomères du 7,9- | 0.86 % | 80.23 % | 1.13 % | 17.78% |

| | | | | |
|---|---|---|---|---|
| dodécadiényl acétate | | | | |
| après 1ère purification à l'urée | 1.33 % | 91.85 % | 1.35% | 5.47 % |
| après 2ème purification à l'urée | 1,88 % | 92.78 % | 0.66 % | 4,68 % |
| après 3ème purification à l'urée | 2,75 % | 91.57 % | 0.35 % | 5.33 % |

On observe qu'un tel traitement à l'urée est long et fastidieux et ne permet pas d'atteindre de très hautes puretés, même en faisant plusieurs purifications à l'urée successives. Ainsi après trois traitements à l'urée successifs suivant le protocole décrit précédemment, on atteint un plafond avec environ 5% d'isomère (E,E), par ailleurs une partie de la phéromone se dégrade.

### Exemple 2 (hors invention) : amélioration de la pureté isomérique d'un mélange d'isomères du 7,9-dodécadiényl acétate par action directe d'un diénophile hydrolysable

On introduit dans un ballon tétracol de 250 mL, 10 g de mélange d'isomères de 7,9-dodécandiényl acétate (44.6 mmol)(pureté isomérique donnée dans le tableau ci-dessous), 40 ml de méthylcyclohexane et 4.37 g d'anhydride maléique (diénophile hydrolysable **D)** (44.6 mmol ; 1.0 équiv.).

Le mélange réactionnel est chauffé à **T=70°C** pendant 3 heures, puis refroidi jusqu'à 30°C, et une solution saturée de NaHCO₃ est ajoutée (1.14 M, 50 ml).

Après 30 minutes sous agitation, la phase aqueuse est extraite à l'heptane (2 x 20 ml), lavée avec une solution aqueuse saturée de NaCl (5.92 M, 20 ml) puis à l'eau (20 mL), et concentrée sous vide.

On obtient 7.5 g du mélange de phéromone purifiée de composition isomérique :

| | Pureté isomérique en % | | | |
|---|---|---|---|---|
| Isomères | (7Z, 9Z) | (7*E*, 9Z) | (7Z, 9E) | (7E, 9E) |

| | | | | |
|---|---|---|---|---|
| 7,9-dodécadiényl acétate avant traitement | 5.59 | 67.47 | 2.82 | 24.10 |
| 7,9-dodécadiényl acétate après traitement | 7.78 | 85.60 | 1.86 | 4.76 |

Cet exemple hors invention illustre deux faits :
1) la réaction Diels Aider ne conduit pas à une élimination totale de l'isomère *(E,E).*
2) le rendement massique de cette étape est de 75% ce qui signifie que l'étape coûteuse précédente (réaction de couplage de l'énolphosphate avec le magnésien) voit sa productivité réelle baisser de 25%.

### Exemple 3 : amélioration de la pureté isomérique d'un mélange d'isomères du diéthyl-hexa-1,3-dién-1-yl phosphate par le procédé selon l'invention conduisant au mélange M1

Un mélange d'isomères du diéthyl-hexa-1,3-diénylphosphate est obtenu à partir du *trans-hexen-1-al* en suivant le procédé décrit dans le brevet WO2016001383.

On introduit dans le réacteur de 20L, 2085 g de ce mélange brut de diéthyl-hexa-1,3-diénylphosphate (8.90 mol) (pureté isomérique donnée dans le tableau ci-dessous), 8.34 L de méthylcyclohexane et 523.0 g d'anhydride maléïque (5.33 mol, soit 0.6 équiv.).

Le mélange réactionnel est chauffé à 70°C pendant 3 heures, puis refroidi à 10°C, et une solution aqueuse d'hydroxyde de sodium est ajoutée (3 M, 4.17L).

Après 30 minutes sous agitation, la phase aqueuse est extraite à l'heptane (2 x 3.13 L), lavée avec une solution aqueuse de NaCl (10% w/w ; 1 x 2.09 L), et concentrée sous vide.

On obtient 1566 g du mélange **M1** de diéthyl-hexa-1.3-diénylphosphate de composition isomérique :

| | Pureté isomérique en % | | | |
|---|---|---|---|---|
| Isomères | (1*Z*, 3E) | (1*Z*, 3Z) | (1 E, 3Z) | (1*E*, 3E) |

| | | | | |
|---|---|---|---|---|
| mélange avant traitement | 1.03 | 1.07 | 73.60 | 24.30 |
| mélange après traitement **M1** | 0.74 | 0.81 | 98.45 | 0 |

### Exemple 4 : obtention du mélange M2 de la phéromone à partir d'un mélange d'isomères du diéthyl-hexa-1,3-dién-1-yl phosphate purifié.

A partir du mélange **M1** de l'énolphosphate de l'exemple 3, on applique le procédé décrit dans WO2016001383A1 pour obtenir le mélange **M2** de phéromone.

On introduit dans le réacteur quadricol, sous argon: le réactif de Grignard alcoolate, préparé à partir de 67.1 g de 6-chloro-hexan-1-ol (491.7 mmol), 474 ml de THF et de magnesium (983,5mmol).

### Le mélange est ensuite refroidi à 5°C puis on ajoute le catalyseur acétyl acétonate de fer (III) (158 mg, 0.1%mol). A ce mélange noir sont additionnés 97.5 g du mélange M1 précédent (416.3 mmol) en 20 minutes et en conservant la température en-dessous de 5°C.

Après 2h d'agitation à 25°C, le mélange est refroidi à -5°C et on ajoute 85 mL d'anhydride acétique goutte à goutte en 20 minutes. Après une heure d'agitation à température ambiante, le mélange réactionnel est acidifié par une solution aqueuse d'HCl (1**N**, 680ml). La phase aqueuse est extraite trois fois avec du méthyl tert-butyl éther (3 x 120ml). Les trois phases organiques sont recombinées, puis lavées à l'eau et concentrées sous vide.

On obtient alors le mélange **M2** de 7,9-dodécandiényl acétate (m = 65.7 g). Le rendement global au départ de l'énolphosphate purifié est de 70% (pureté massique = 95% ; pureté isomérique = 98%).

| | Pureté isomérique en % | | | |
|---|---|---|---|---|
| Isomères | (1*Z*, 3E) | (1*Z*, 3Z) | (1 E, 3Z) | (1*E*, 3E) |
| Mélange **M1** | 0.74 | 0.81 | 98.45 | 0 |
| Isomères | (7Z, 9*Z*) | (7E, 9Z) | (7Z, 9E) | (7E, 9E) |
| Mélange **M2** | | 98.0 | | 0.9 |

## Revendications

1. Procédé de préparation d'un mélange **M1** d'isomères d'énolphosphate de formule **1** 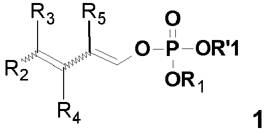 dans laquelle R₁ et désignent indépendamment un groupement alkyle comprenant de 1 à 6 atomes de carbone ou un groupement aryle, R₂ est un groupe alkyle linéaire comprenant de 1 à 8 atomes de carbone, R₃, R₄ et R₅ sont choisis indépendamment parmi H et CH₃, exempt d'isomère (E,E) et comprenant au moins 98% d'isomère (E,Z), au moins 0.1% d'isomère (Z,Z) et au moins 0.1% d'isomère (Z,E) comprenant les étapes de :
a) Mise en contact d'un mélange d'isomères d'énolphosphate de formule **1** comprenant une quantité détectable d'isomère (E,E) avec un diénophile hydrolysable **D** de préférence dans un solvant organique **S,** à une température **T,** et
b) Hydrolyse basique du milieu obtenu et élimination de l'adduit formé pour obtenir le mélange **M1,**
c) **caractérisé en ce qu'**une quantité détectable est une quantité supérieure à 0.10%,
d) et que le diénophile hydrolysable est choisi dans le groupe consistant en l'acide maléique, l'acide acrylique, l'acide méthacrylique ainsi leurs dérivés hydrolysables choisis parmi leurs esters ou anhydrides.

2. Procédé selon la revendication 1, dans lequel Le mélange d'isomères d'énolphosphate comprenant une quantité détectable d'isomère (E,E) est obtenu à partir du (E) hex-2-énal.

3. Procédé pour obtenir un mélange **M2** des isomères d'un composé de formule **3** 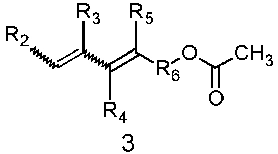 dans laquelle R₂ est un groupe alkyle linéaire comprenant de 1 à 8 atomes de carbone, R₃, R₄ et R₅ sont choisis indépendamment parmi H et CH₃, R₆ représente un groupement alkyle linéaire ou ramifié C₁-C₆ , **caractérisé en ce que** sa pureté isomérique est supérieure ou égale à 98% en l'isomère (E,Z) et qu'il contient au moins 0.1% des isomères (Z,E) et (Z,Z) et moins de 1% de l'isomère (E,E), comprenant les étapes selon la revendication 1 ou 2, et comprenant en outre une étape c) de mise en contact du mélange **M1** d'isomères d'énolphosphate de formule **1** exempt d'isomère (E,E) et comprenant au moins 98% d'isomère (E,Z), au moins 0.1% d'isomère (Z,Z) et au moins 0.1% d'isomère (Z,E) obtenu à l'étape b) avec un composé de formule XMg-R₆-OMgX, dans laquelle chaque X représente indépendamment un atome d'halogène, et R₆ représente un groupement alkyle linéaire ou ramifié C₁-C₆, puis la mise en contact du mélange obtenu avec un agent d'acylation qui est choisi dans le groupe comprenant les halogénures d'acétoyle, l'anhydride acétique et les acétates d'alkyle tel que l'acétate d'éthyle.

4. Procédé selon l'une quelconque des revendications 1 à 3, dans lequel l'énolphosphate de formule 1 est un composé de formule **2** 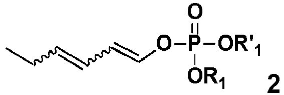 R₁ et R'₁ ayant la même signification que dans la revendication 1.

5. Procédé selon l'une quelconque des revendications 1 à 4, dans lequel la température T est égale à 70°C.

6. Procédé selon l'une quelconque des revendications 1 à 5, dans lequel le diénophile hydrolysable **D** est l'anhydride maléique.

7. Procédé selon l'une quelconque des revendications 1 à 6, dans lequel le solvant organique S est le méthylcyclohexane.

8. Mélange **M1** des isomères (E,Z), (Z,E), et (Z,Z) d'un énolphosphate de formule **1** tel que défini dans la revendication 1, **caractérisé en ce que** sa pureté isomérique est supérieure ou égale à 98% en l'isomère (E,Z) et qu'il contient au moins 0.1% des isomères (Z,E) et (Z,Z).

9. Mélange **M1** selon la revendication 8, dans lequel l'énolphosphate de formule **1** est le diéthyl-hexa-1,3-dién-1-yl phosphate.

## Patentansprüche

1. Verfahren zur Herstellung eines Enolphosphatisomer-Gemischs M1 der Formel 1 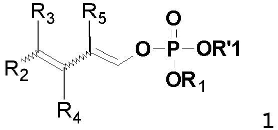 wobei R₁ und R'₁ unabhängig eine Alklylgruppe mit 1 bis 6 Kohlenstoffatomen oder eine Arylgruppe bezeichnen, R₂ eine lineare Alkylgruppe mit 1 bis 8 Kohlenstoffatomen ist, R₃, R₄ und R₅ unabhängig aus H und CH₃ ausgewählt werden, ohne Isomer (E,E), und umfassend mindestens 98% Isomer (E,Z), mindestens 0,1% Isomer (Z,Z) und mindestens 0,1% Isomer (Z,E), umfassend die folgenden Schritte:
a) Inkontaktversetzen eines Enolphosphatisomer-Gemischs der Formel 1, das eine detektierbare Menge an Isomer (E,E) umfasst, mit einen hydrolysierbaren Dienophil D, vorzugsweise in einem organischen Lösungsmittel S, bei einer Temperatur T, und
b) basische Hydrolyse des erhaltenen Mediums und Entfernen des gebildeten Addukts, um das Gemisch M1 zu erhalten,
c) **dadurch gekennzeichnet, dass** eine detektierbare Menge eine Menge über 0,10% ist,
d) und dass das hydrolysierbare Dienophil aus der Gruppe ausgewählt ist, die aus Maleinsäure, Acrylsäure, Methacrylsäure sowie deren hydrolysierbaren Derivaten besteht, ausgewählt aus deren Estern oder Anhydriden.

2. Verfahren nach Anspruch 1, wobei das Enolphosphatisomer-Gemisch, das eine detektierbare Menge an Isomer (E,E) umfasst, aus (E) Hex-2-enal gewonnen wird.

3. Verfahren zur Gewinnung eines Gemischs M2 der Isomere einer Verbindung der Formel 3 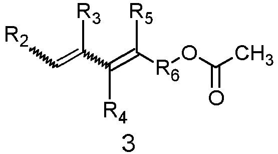 wobei R₂ eine lineare Alkylgruppe mit 1 bis 8 Kohlenstoffatomen ist, R₃, R₄ und R₅ unabhängig aus H und CH₃ ausgewählt werden, R₆ eine lineare oder verzweigte C₁-C₆-Alkylgruppe darstellt, **dadurch gekennzeichnet, dass** seine Isomerreinheit größer oder gleich 98% an Isomer (E,Z) ist und dass es mindestens 0,1% der Isomere (Z,E) und (Z,Z) und weniger als 1% des Isomers (E,E) enthält, umfassend die Schritte nach Anspruch 1 oder 2, und umfassend ferner einen Schritt c) des Inkontaktversetzens des Enolphosphatisomer-Gemischs M1 der Formel 1 ohne Isomer (E,E) und umfassend mindestens 98% Isomer (E,Z), mindestens 0,1% Isomer (Z,Z) und mindestens 0,1% Isomer (Z,E), erhalten in Schritt b), mit einer Verbindung der Formel XMg-R₆-OMgX, wobei jedes X unabhängig ein Halogenatom darstellt, und R₆ eine lineare oder verzweigte C₁-C₆-Alkylgruppe darstellt, dann das Inkontaktversetzen des erhaltenen Gemischs mit einem Acylationsmittel, das aus der Gruppe ausgewählt ist, die die Acetoylhalogenide, Acetanhydrid und die Alkylacetate wie Ethylacetat umfasst.

4. Verfahren nach einem der Ansprüche 1 bis 3, wobei das Enolphosphat der Formel 1 eine Verbindung der Formel 2 ist, 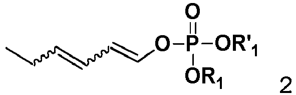 wobei R₁ und R'₁ dieselbe Bedeutung wie in Anspruch 1 haben.

5. Verfahren nach einem der Ansprüche 1 bis 4, wobei die Temperatur T gleich 70°C ist.

6. Verfahren nach einem der Ansprüche 1 bis 5, wobei das hydrolysierbare Dienophil D Maleinsäure ist.

7. Verfahren nach einem der Ansprüche 1 bis 6, wobei das organische Lösungsmittel S Methylcyclohexan ist.

8. Gemisch M1 der Isomere (E,Z), (Z,E) und (Z,Z) eines Enolphosphats der Formel 1 nach Anspruch 1, **dadurch gekennzeichnet, dass** seine Isomerreinheit über oder gleich 98% an dem Isomer (E,Z) beträgt und dass es mindestens 0,1% der Isomere (Z,E) und (Z,Z) enthält.

9. Gemisch M1 nach Anspruch 8, wobei das Enolphosphat der Formel 1 Diethyl-hexa-1,3-dien-1-yl-phosphat ist.

## Claims

1. Preparation process for a mixture M1 of enol phosphate isomers of formula 1 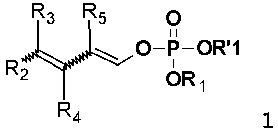 wherein R₁ and independently designate an alkyl group comprising 1 to 6 carbon atoms or an aryl group, R₂ is a linear alkyl group comprising 1 to 8 carbon atoms, R₃, R₄ and R₅ are chosen independently from H and CH₃, free of (E,E) isomer and comprising at least 98% of (E,Z) isomer, at least 0.1% of (Z,Z) isomer and at least 0.1% of (Z,E) isomer, comprising the steps of:
a) Contacting a mixture of enol phosphate isomers of formula 1 comprising a detectable quantity of (E,E) isomer with a hydrolysable dienophile D, preferably in an organic solvent S, at a temperature T, and
b) Basic hydrolysis of the medium obtained and elimination of the adduct formed to obtain mixture M1,
c) **characterized in that** a detectable quantity is a quantity greater than 0.10%,
d) and that the hydrolysable dienophile is chosen from the group consisting of maleic acid, acrylic acid, methacrylic acid, and their hydrolysable derivatives chosen from their esters or anhydrides.

2. Process according to claim 1, wherein the enol phosphate isomer mixture comprising a detectable quantity of (E,E) isomer is obtained from (E) hex-2-enal.

3. Process to obtain an isomer mixture M2 of a compound of formula 3 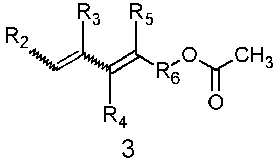 wherein R₂ is a linear alkyl group comprising 1 to 8 carbon atoms, R₃, R₄ and R₅ are independently chosen from among H and CH₃, R₆ represents a linear or branched C₁-C₆ alkyl group, **characterized in that** its isomeric purity is greater than or equal to 98% in (E,Z) isomer and **in that** it contains at least 0.1% of (Z,E) and (Z,Z) isomers and less than 1% of (E,E) isomer, comprising the steps according to claim 1 or 2, and further comprising a step c) of contacting the mixture M1 of enol phosphate isomers of formula 1 free of (E,E) isomer and comprising at least 98% of isomer (E,Z), at least 0.1% of (Z,Z) isomer and at least 0.1% of (Z,E) isomer obtained at step b) with a compound of formula XMg-R₆-OMgX, wherein each X independently represents a halogen atom, and R₆ represents a linear or branched C₁-C₆ alkyl group, then contacting the mixture obtained with an acylation agent which is chosen from the group comprising acetoyl halides, acetic anhydride and alkyl acetates such as ethyl acetate

4. Process according to any one of claims 1 to 3, wherein the enol phosphate of formula 1 is a compound of formula 2 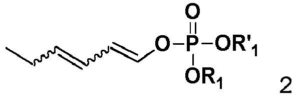 R₁ and R'₁ having the same meaning as in claim 1.

5. Process according to any one of claims 1 to 4, wherein the temperature T is equal to 70°C.

6. Process according to any one of claims 1 to 5, wherein the hydrolysable dienophile D is maleic anhydride.

7. Process according to anyone of claims 1 to 6, wherein the organic solvent S is methylcyclohexane.

8. Mixture M1 of (E,Z), (Z,E), and (Z,Z) enol phosphate isomers of formula 1 as defined in claim 1, **characterized in that** its isomeric purity is greater than or equal to 98% in (E,Z) isomer and that it contains at least 0.1% of (Z,E) and (Z,Z) isomers.

9. Mixture M1 according to claim 8, wherein the enol phosphate of formula 1 is diethyl-hexa-1,3-dien-1-yl phosphate.
